# EUROPEAN PATENT APPLICATION

(11) **EP 0 049 968 A1**
(43) Date of publication of application: **21.04.1982**
(21) Application number: 81304455.9
(22) Date of filing: 28.09.1981
(51) Int. Cl.: A61K 31/43

(54) **Pharmaceutical compositions**

(30) Priority: 15.10.1980 GB 8033273; 12.11.1980 GB 8036387; 22.06.1981 GB 8119185
(71) Applicant: BEECHAM GROUP PLC, Brentford, Middlesex TW8 9BD (GB)
(72) Inventor: Hunter, Pamela Ann, Horsham Sussex (GB)
(74) Representative: Hesketh, Alan (GB)

(57) **Abstract**

A pharmaceutical composition which comprises the compound of formula (I) or a pharmaceutically acceptable salt or in vivo hydrolysable ester thereof: 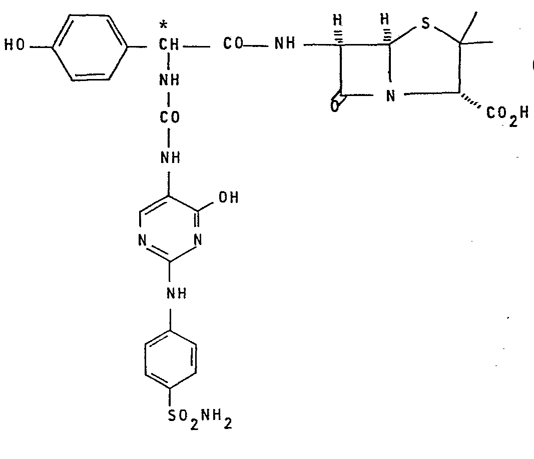 and a compound of formula (II) or a pharmaceutically acceptable salt or ester thereof: 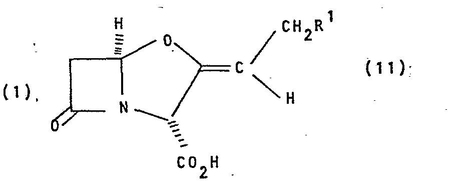 wherein R¹ is hydrogen, hydroxy, substituted alkyl, substi- uted hydroxy, thiol, substituted thiol, substituted sulphonyl, ubstituted sulphinyl, amino, mono- or di-hydrocarbyl sub- tituted amino, or mono- or di-acylamino.

## Description

The present invention relates to pharmaceutical compositions. In particular the invention relates to pharmaceutical compositions for treating bacterial infections in mammals including man.

European Patent Publication 0003814 discloses a class of novel penicillins of general formula (A) or salts thereof: 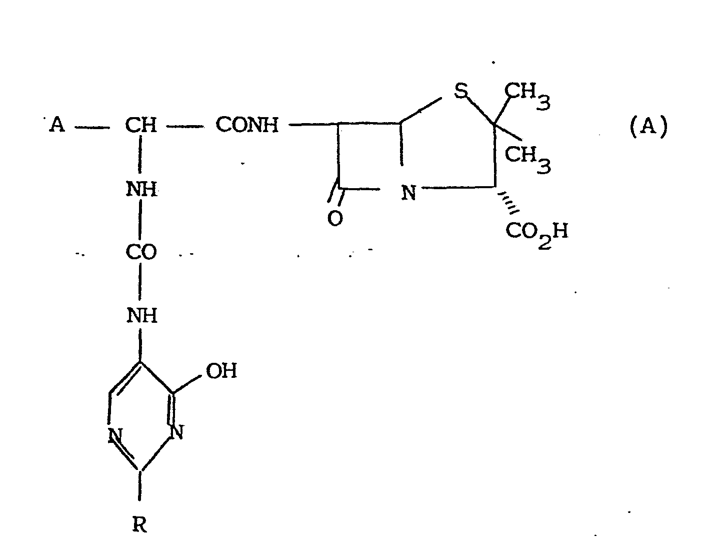 wherein A represents an optionally substituted phenyl group, a thienyl, cyclohexyl, cyclohexenyl or cyclohexa- dienyl group; and R represents hydrogen, an alkyl, alkenyl, cycloalkyl or cycloalkenyl group, an optionally substituted phenyl, a cyclopropyl alkyl group, a hydroxy group optionally substituted by alkyl, alkenyl, cycloalkyl, phenyl or benzyl, an optionally substituted mercapto group, an alkylsulphenyl group, a free or substituted amino group, an optionally substituted piperazino or phenylalkylamino group, an acylamino group or an alkyl, aralkyl or arylsulphonyl amino group.

It has now been discovered that when a compound from within the large class of compounds of formula (A) is formulated with clavulanic acid or a clavulanic acid derivative or a pharmaceutically acceptable salt or ester thereof then the resulting co-formulation has a particularly high activity against a wide range of bacteria.

Accordingly, the present invention provides a pharmaceutical composition which comprises the compound of formula (I) or a pharmaceutically acceptable salt or in vivo hydrolysable ester thereof: 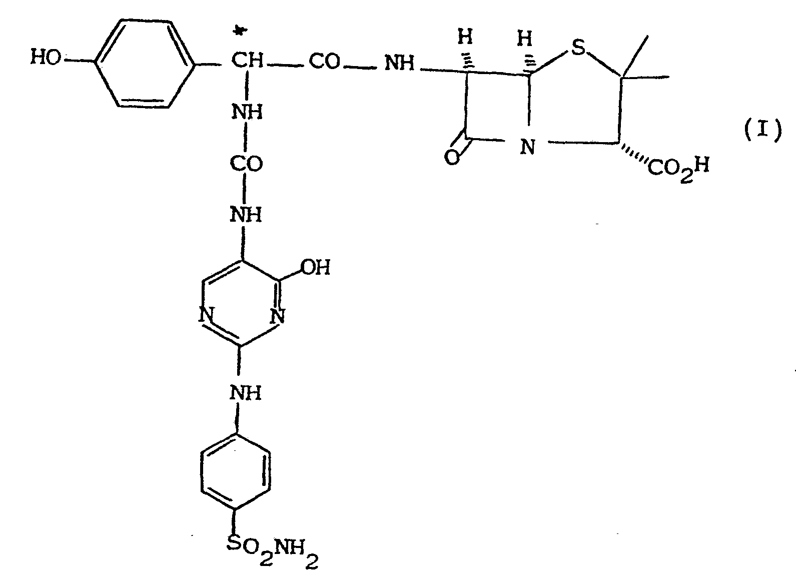 and a compound of formula (II) or a pharmaceutically acceptable salt or ester thereof: 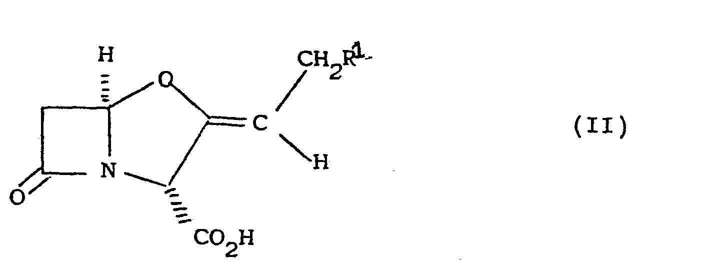 wherein R is hydrogen, hydroxy, substituted alkyl, substituted hydroxy, thiol, substituted thiol, substituted sulphonyl, substituted sulphinyl, amino, mono or di-hydrocarbyl substituted amino, or mono or di-acylamino.

The carbon atom marked ^{*} in formula (I) is asymmetric and the compound may be derived from the side-chain having a D, L or DL configuration at that position. All forms of compound (I) are included in this invention. Suitably, the carbon atom marked ^{*} is derived from the D-oonfiguration and is conveniently referred to as the D-penicillin.

Suitable salts of the compounds of formulae (I) and (II) include metal salts e.g. aluminium, alkali metal salts such as sodium or potassium alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalky- lamines such as bicyclohexylamine, or with procaine, dibenzylamine, N,N-dibenzylethylenediamine, 1-ephenamine, N-ethylpiperidine, N-benzyl-P-phenethylamine, dehydroabietylamine, N,N'-bisdehydroabietylamine ethylenediamine, or other amines which have been used to form salts with known penicillins.

Suitable in vivo hydrolysable esters of the compound of formula (I) include, for example acyloxyalkyl groups such as acetoxymethyl, pivaloyloxymethyl, a-acetoxyethyl and a-pivaloyloxyethyl groups; alkoxycarbonyloxyalkyl groups, such as ethoxycarbonyloxymethyl and a-ethoxycarbonyloxyethyl; dialkylaminoalkyl groups such as dimethylaminomethyl, dimethylaminoethyl, diethylaminomethyl or diethylaminoethyl; and lactone groups such as phthalidyl or dimethoxyphthalidyl.

Preferred groups R¹ include C₁₋₆ alkyloxy, C₁₋₆ alkylthio, C₁₋₆ alkyl sulphoxide, C₁₋₆ alkenylsulphoxide, C₁₋₆ alkenylsulphone, C₁₋₆ alkylsulphone, amino C₁₋₆ alkyloxy, C₁₋₆ alkenyloxy, Cₗ₋₆ alkyloxyalhyloxy, amino, Cₗ₋₆ alkylamino, di-C₁₋₆ alkylamino, aralkylamino, C₁₋₆ alkanoylamino or di-C₁₋₆ alkanoylamino, pentan-2,4-dion-3-yl.

A further preferred group R¹ is hydroxy.

Most suitably R¹ is methoxy, ethoxy, propoxy, butoxy, methylthio, ethylthio, acetamidoethenylsulphone, acetamidapropenylsulphone, acetamidoethylsulphone, acetamidomethylsulphone, methylsulphone, ethylsulphone, thiocarbamate, 2-aminoethoxy, 3-aminqpropoxy, 2-amino- propoxy, vinyloxy, methoxymethoxy, ethoxymethoxy, methoxyethoxy, ethoxyethoxy, amino, methylamino, ethylamino, propylamino, butylamino, benzylamino, diethylamino, dibutylamino, acetylamino or diacetylamino.

One subgroup of compounds of the formula (II) comprises the compounds wherein R¹ is hydrogen, substituted alkyl, substituted hydroxy, thiol, substituted thiol, substituted sulphonyl, substituted sulphinyl, amino, mono or di-hydrocarbyl substituted amino, or mono or di-acylamino.

Specific compounds of formula (II) which may advantageously be formulated with compound (I) include: clavulanic acid (R¹=OH) or a salt or ester thereof; 9-O-methylclawlanic acid (R¹=OCH₃) or a salt or ester thereof; 9-N-isobutylaminodeoxyclavulanic acid (R¹=NHCH₂CH(CH₃)₂) or an ester thereof.

Preferred salts of clavulanic acid are the sodium and potassium salts. Preferred salts of the methyl ether (R=OCH₃) are the sodium, potassiun and calcidium salts. The isobutyl amine (R¹NHCH₂CH(CH₃)₂) is preferably in the form of a zwitterion.

In general the compositions of this invention will contain the compounds of formulae (II) and (I) in the ratio of from 5:1 to 1:25 respectively. More suitably this ratio will be fran 1:1 to 1:15 and preferably from 1:2 to 1:12, for example from 1:3 to 1:10, respectively.

The compositions of this invention may be presented as single or multi dose forms. In general single dose forms will contain from 62.5 mg to 2500 mg of the penicillin and from 25 mg to 250 mg of the synergist of formula (II). These compositions may be administered once or more times a day so that for a 70 kg human the usual total daily dose of penicillin administered is from 250 mg to 5000 mg and the usual daily dose of synergist is from 100 mg to 1000 mg.

It is possible to prepare the compositions of this invention by conventional methods of mixing and formulation, for example, as described in British Patent No. 1508977. Accordingly, the present invention also provides a process for the preparation of a pharmaceutical composition as hereinbefore described which comprises bringing into association the components of said composition. A particularly suitable form of the composition comprises a mixture of the compound of formula (I) and the compound of formula (II) sealed into glass vials or bottles. Naturally the injectable compositions of this invention are rendered sterile.

The composition of this invention will be dissolvable in conventional solvents such as, for example, water for injection.

The compositions of this invention may be employed for the treatment of bacterial infections due to a wide range of gram-negative and gram-positive bacteria such as Pseudomonas aeruginosa, Klebsiella aerogenes, Escherichia coli, Staphylococcus aureus, Serratia marcescens and Bacteroides fragilis, including those which produce 8- lactamases.

Normally and preferably the active compounds are administered in a composition of this invention as hereinbefore described. Most suitably the compositions are administered to provide a peak blood level of at least 0.25 µg/ml, more suitably at least 0.5 µg/ml and preferably at least 1 µg/ml of the compound of the formula (II) or salt thereof and at least 1 µg/ml, more suitably at least 2.5 µg/ml and preferably at least 12.5 ug/ml of a compound of the formula (I).

The compounds of the formula (II) may be prepared by the methods of British Patents Nos 1508977, 1508978, 1565209, 1566706, 1574906, 1543467, 1593275 and Belgian Patents Nos 861325, 861716, 866276 866496 and 870405.

The toxicity of the compositions of this invention towards mammals, including man, is no greater than the sum of the toxicities of the individual components of the compositions.

The following Examples illustrate the invention:

### Example 1

Compounds (i) and (ii): 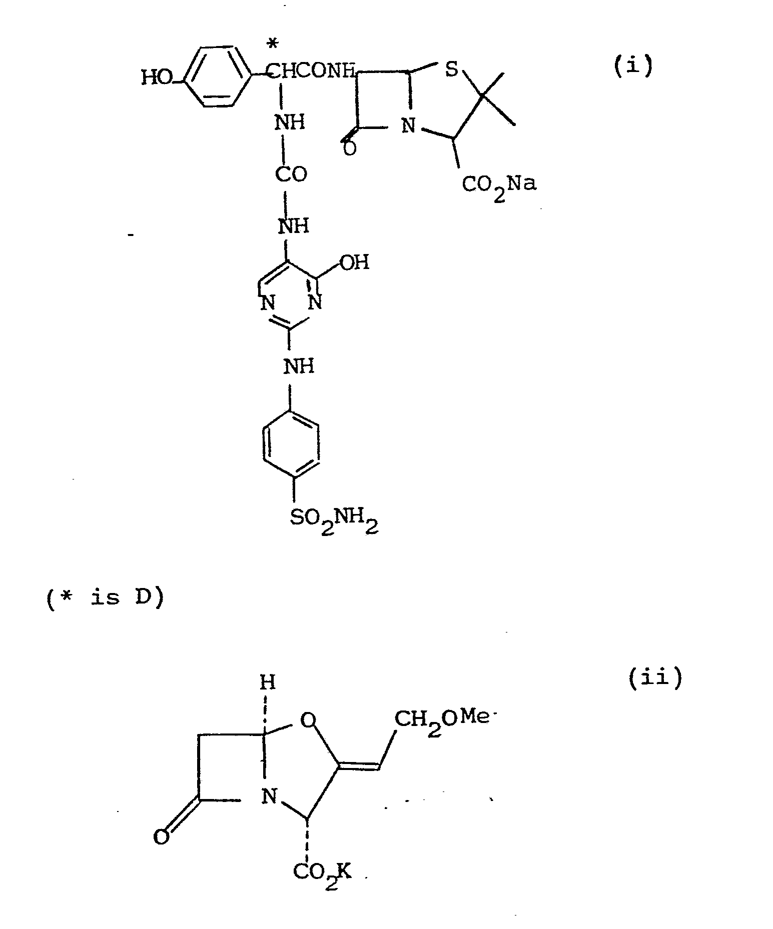 in sterile finely divided form may be filled into sterile glass vials in the following quantities:

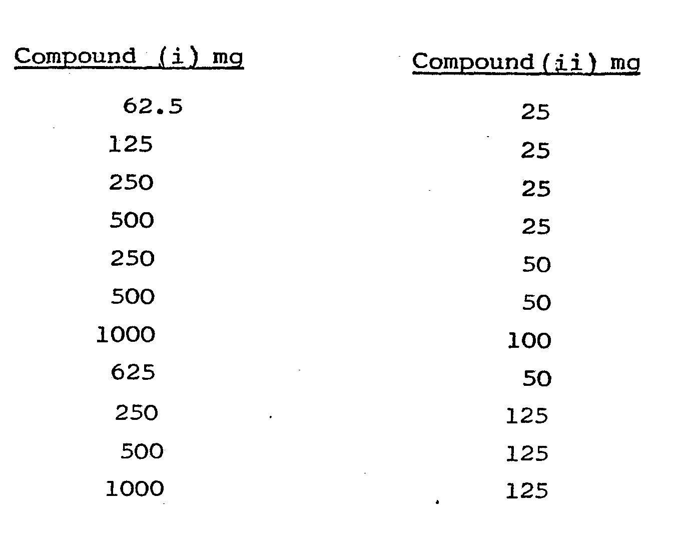

### Example 2

Compounds (i) and Compound (iii):

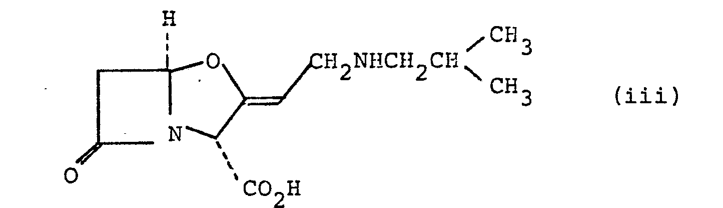

in sterile finely divided form may be filled into sterile glass vials in the following quantities:

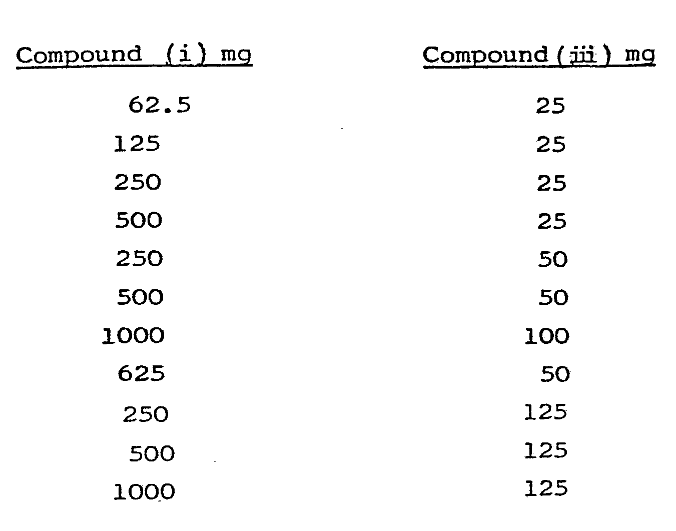

### Biological Data

Activity of compound (i) alone and in combination with compound (iii) ~ K-aerogenes strains producing both a plasmid-mediated and chromosomally mediated β-lactamase

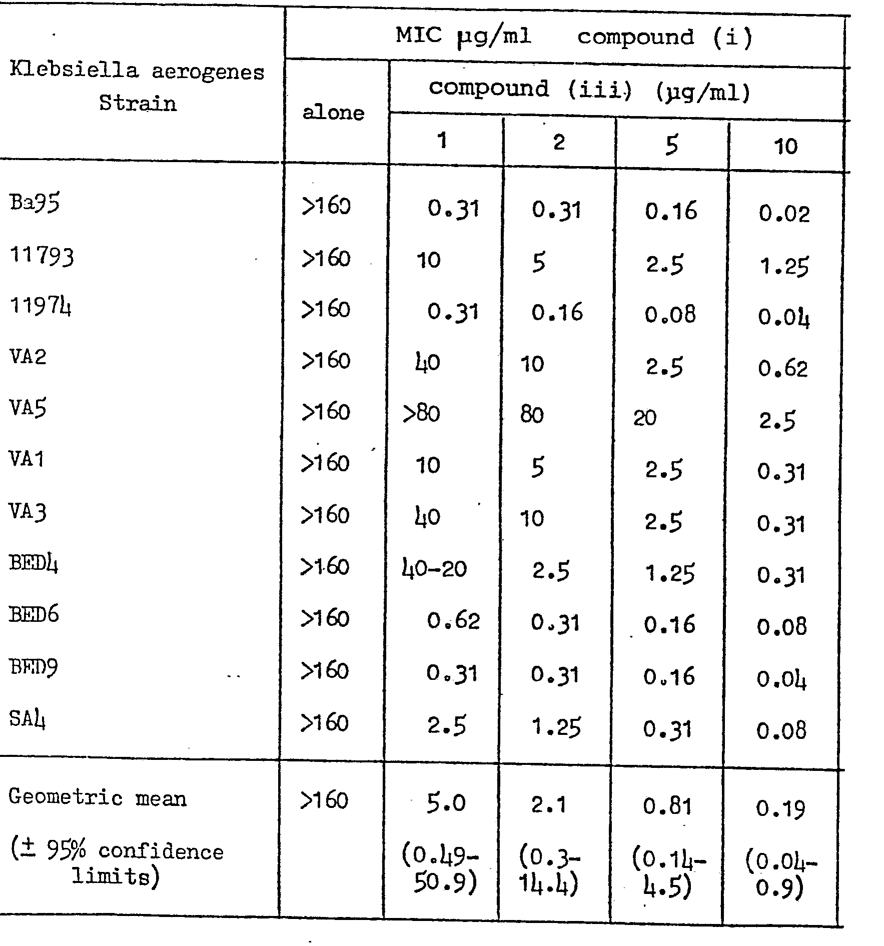

Activity of compound (i) alone and in combination with compound (iii) against highly resistant E.coli strains (producing a plasmid mediated β-lactamase)

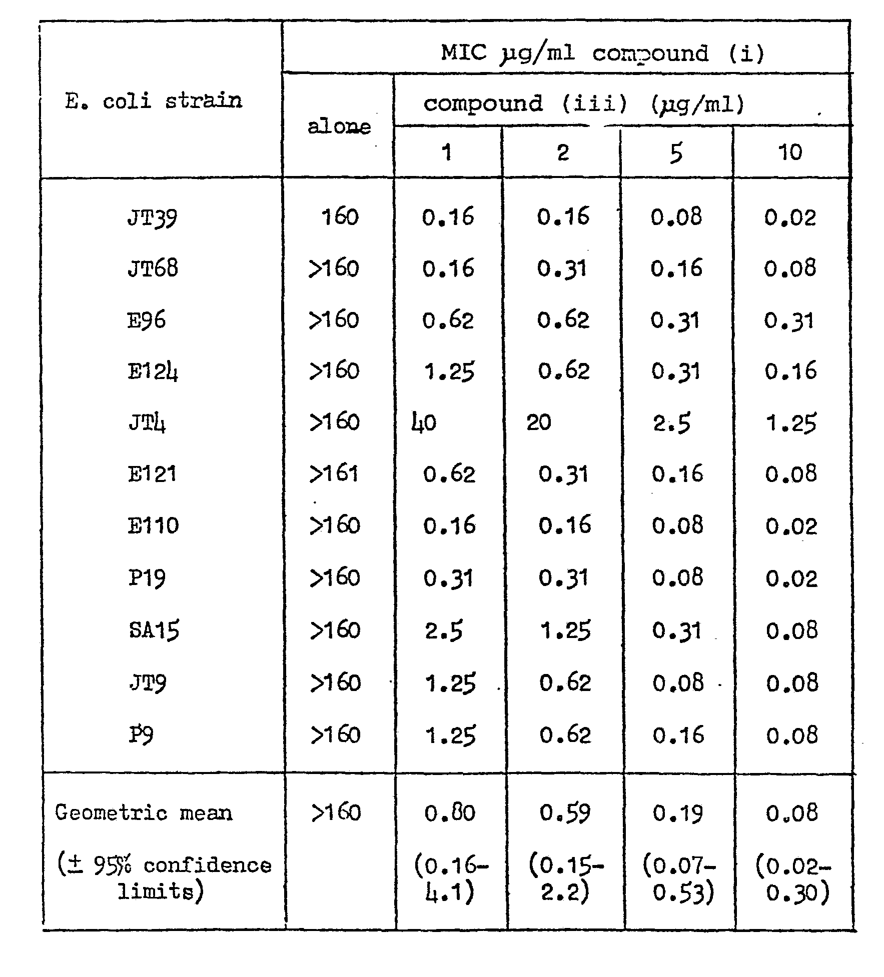

Activity of compound (i) alone and in combination with compound (iii) against highly resistant strains of Pseudomonas aeruginosa (producing both a plasmid-and chromosomally-mediated β-lactamase)

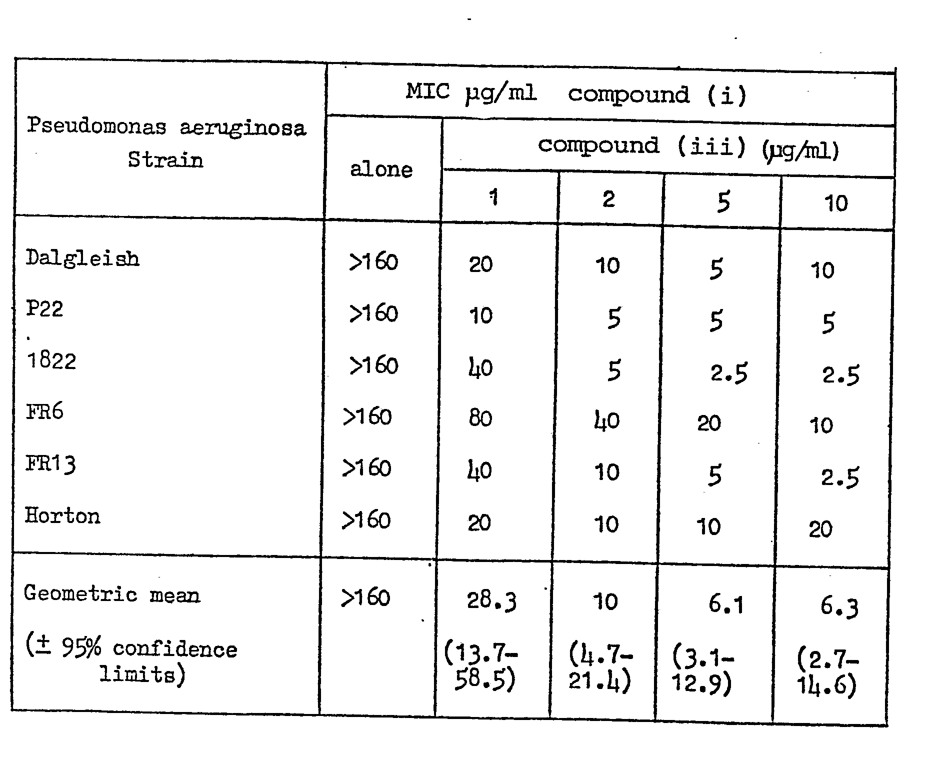

### Example 3

Compounds (i) and (iv): 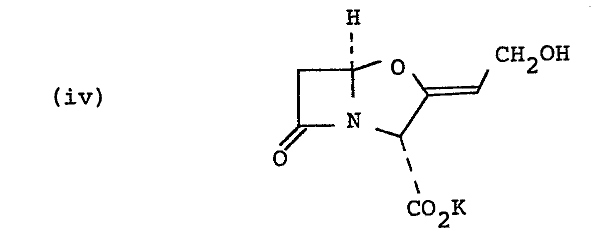 in sterile finely divided form may be filled into sterile glass vials in the following quantities:

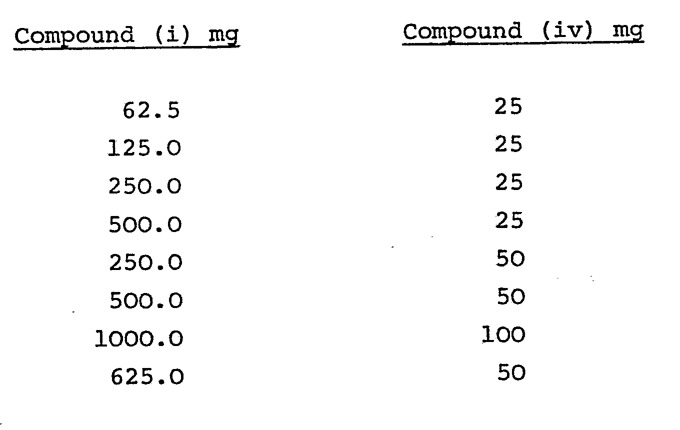

### Biological Data

Activities of the compound (i) against a number of pathogenic organisms alone, and in the presence of 1.0, 2.5, 5.0 and 10 µg/ml of compound (iv).

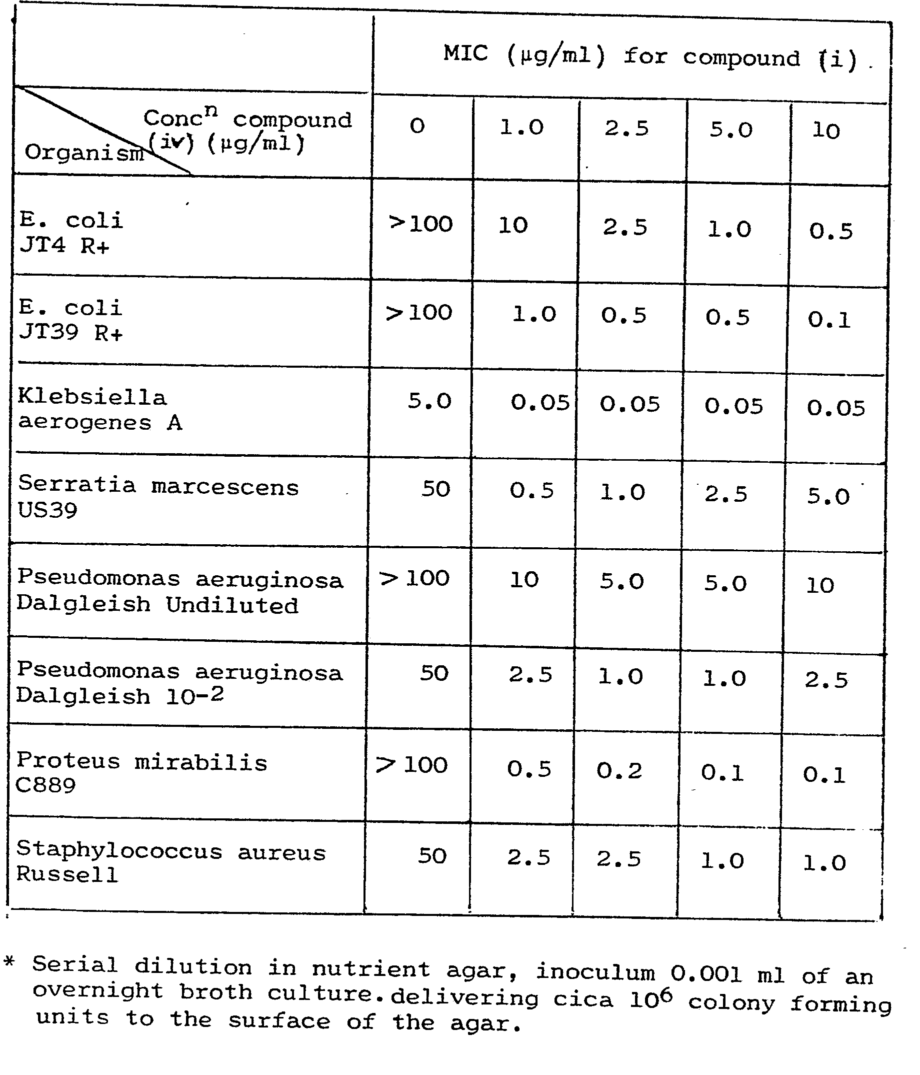

## Claims

1. A pharmaceutical composition which comprises the compound of formula (I) or a pharmaceutically acceptable salt or in vivo hydrolysable ester thereof: 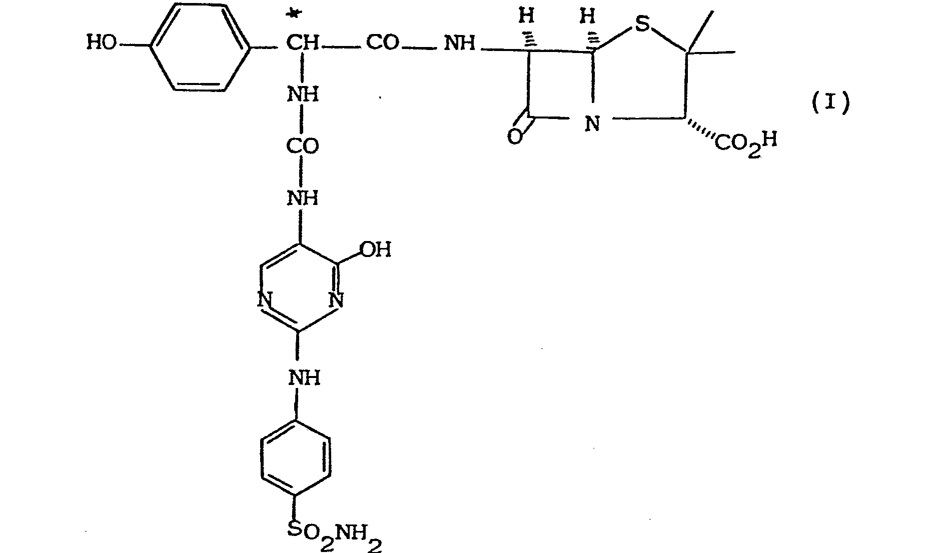 and a compound of formula (II) or a pharmaceutically acceptable salt or ester thereof: 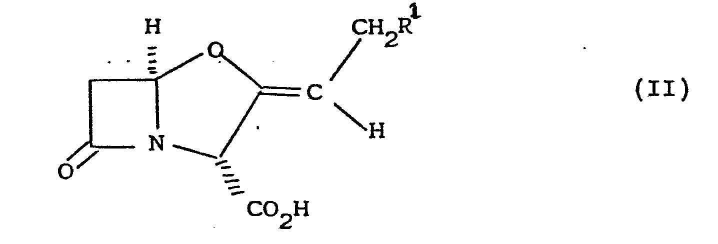 wherein R¹ is hydrogen, hydroxy, substituted alkyl, substituted hydroxy, thiol, substituted thiol, substituted sulphonyl, substituted sulphinyl, amino, mono- or di-hydrocarbyl substituted amino, or mono- or di-acylamino.

2. A pharmaceutical composition as claimed in claim 1 wherein R¹ is hydrogen, substituted alkyl, substituted hydroxy, thiol, substituted thiol, substituted sulphonyl, substituted sulphinyl, amino, mono or di-hydrocarbyl substituted amino, or mono or di-acylamino.

3. A pharmaceutical composition as claimed in claim 1 or claim 2 wherein the carbon atom marked ^{*} in formula (I) is in the D configuration.

4. A pharmaceutical composition as claimed in claim 1 or claim 2 wherein R¹ is C₁₋₆ alkyloxy, C₁₋₆ alkylthio, C₁₋₆ alkyl sulphoxide, C₁₋₆ alken^{y}lsul^{p}hoxide, C₁₋₆ alkenylsulphone, C₁₋₆ alkylsulphone, amino C₁₋₆ alkyloxy, C₁₋₆ alkenyloxy, C_{1-6 alk}yₗₒₓyₐₗₖyₗₒₓy , amino, C₁₋₆ alkylamino, di-C₁₋₆ alkylamino, aralkylamino, C₁₋₆ alkanoylamino or di-C₁₋₆ alkanoylamino, pentan-2,4-dion-3-yl.

5. A pharmaceutical composition as claimed in claim 4 wherein R¹ is methoxy, ethoxy, propoxy, butoxy, methylthio, ethylthio, acetamidoethenylsulphone, acetamidopropenylsulphone, acetamidoethylsulphone, acetamidomethylsulphone, methylsulphone, ethylsulphone, thiocarbamate, 2-aminoethoxy, e-aminopropoxy, 2-aminopropoxy, vinyloxy, methoxymethoxy, ethoxymethoxy, methoxyethoxy, ethoxyethoxy, amino, methylamino, ethylamino, propylamino, butylamino, benzylamino, diethylamino, dibutylamino, acetylamino or diacetylamino.

6. A pharmaceutical composition as claimed in claim 1 or claim 2 wherein R¹ is hydroxy.

7. A pharmaceutical composiiton as claimed in any one of claims 1 to 5 wherein R^{l} is methoxy.

8. A pharmaceutical composition as claimed in any one of claims 1 to 5 wherein R¹ is iso-butyl amino.

9. A process for the preparation of a pharmaceutical composition as claimed in any one of claims 1 to 8 which comprises bringing into association the components of said composition.

10. A pharmaceutical composition substantially as hereinbefore described with reference to the Examples.

11. A compound of formula (I) and a compound of formula (II) as defined in claim 1 for use in the treatment of bacterial infections.

12. A method of treatment of bacterial infections which comprises administration of a compound of formula (I) and a compound of formula (II) as defined in claim 1.

1. A process for the preparation of a a pharmaceutical composition which comprises the compound of formula (I) or a pharmaceutically acceptable salt or in vivo hydrolysable ester thereof: 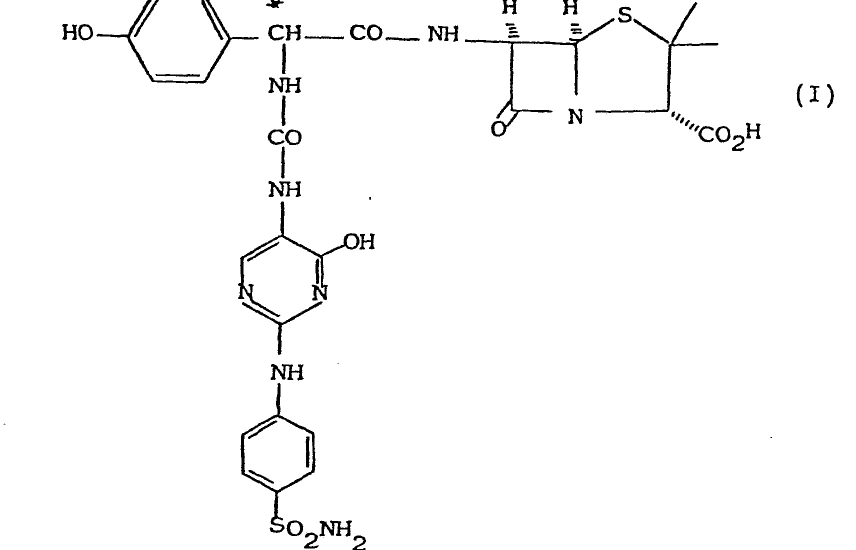 and a compound of formula (II) or a pharmaceutically acceptable salt or ester thereof: 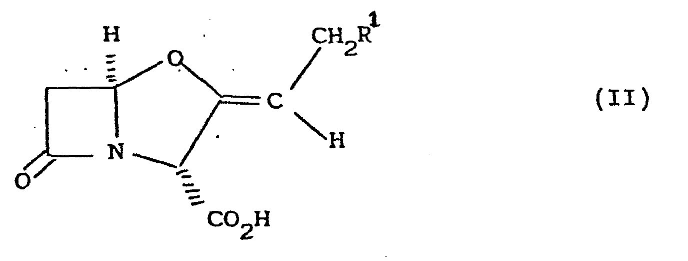 wherein R¹ is hydrogen, hydroxy, substituted alkyl, substituted hydroxy, thiol, substituted thiol, substituted sulphonyl, substituted sulphinyl, amino, mono- or di-hydrocarbyl substituted amino, or mono- or di-acylamino which process comprises bringing into association the components of said composition.

^{2.} A process as claimed in claim 1 wherein R is hydrogen, substituted alkyl, substituted hydroxy, thiol, substituted thiol; substituted sulphonyl, substituted sulphinyl, amino, mono or di-hydrocarbyl substituted amino, or mono or di-acylamino.

3. A process as claimed in claim 1 or claim 2 wherein the carbon atom marked ^{*} in formula (I) is in the D configuration.

4. A process as claimed in claim 1 or claim 2 wherein R¹ is C₁₋₆ alkyloxy, C₁₋₆ alkylthio, C₁₋₆ alkyl sulphoxide, C₁₋₆ alkenylsulphoxide, C₁₋₆ alkenylsulphone, C₁₋₆ alkylsulphone, amino C₁₋₆ alkyloxy, C₁₋₆ alkenyloxy, C_{1-6 alk}yₗₒₓyₐₗₖyₗₒₓy amino, C₁₋₆ alkylamino, di-C₁₋₆ alkylamino, aralkylamino, Cₗ₋₆ alkanoylamino or di-C₁₋₆ alkanoylamino, pentan-2,4-dion-3-yl.

5. A process as claimed in claim 4 wherein R¹ is methoxy, ethoxy, propoxy, butoxy, methylthio, ethylthio, acetamidoethenylsulphone, acetamidopropenylsulphone, acetamidoethylsulphone, acetamidomethylsulphone, methylsulphone, ethylsulphone, thiocarbamate, 2-aminoethoxy, e-aminopropoxy, 2-aminopropoxy, vinyloxy, methoxymethoxy, ethoxymethoxy, methoxyethoxy, ethoxyethoxy, amino, methylamino, ethylamino, propylamino, butylamino, benzylamino, diethylamino, dibutylamino, acetylamino or diacetylamino.

6. A process as claimed in claim 1 or claim 2 wherein R¹ is hydroxy.

7. A process . as claimed in any one of claims 1 to 5 wherein R¹ is methoxy.

8. A process as claimed in any one of claims 1 to 5 wherein R¹ is iso-butyl amino.
